# FASCICULE DE BREVET EUROPEEN

(11) **EP 2 566 842 B1**
(45) Date de publication et mention de la délivrance du brevet: **04.05.2016**
(21) Numéro de dépôt: 11733990.3
(22) Date de dépôt: 09.05.2011
(51) Int. Cl.: C07C 227/18, C07C 229/08, C07C 227/04

(54) **PROCEDE DE PREPARATION D'AMINO-ACIDES OU ESTERS SATURES COMPRENANT UNE ETAPE DE METATHESE**
VERFAHREN ZUR HERSTELLUNG VON GESÄTTIGTEN AMINOSÄUREN ODER GESÄTTIGTEN AMINOESTERN MIT EINEM METATHESESCHRITT
PROCESS FOR PREPARING SATURATED AMINO ACIDS OR SATURATED AMINO ESTERS COMPRISING A METATHESIS STEP

(30) Priorité: 07.05.2010 FR 1053595
(43) Date de publication de la demande: 13.03.2013
(73) Titulaire: Arkema France, 92700 Colombes (FR); Université de Rennes I, 35065 Rennes Cedex (FR); Centre National de la Recherche Scientifique, 75016 Paris (FR)
(72) Inventeur: COUTURIER, Jean-Luc, F-69006 Lyon (FR); DUBOIS, Jean-Luc, F-69390 Millery (FR); MIAO, Xiaowei, F-75011 Paris (FR); FISCHMEISTER, Cedric, F-35630 Vignoc (FR); BRUNEAU, Christian, F-35235 Thorigne Fouillard (FR); DIXNEUF, Pierre, F-35000 Rennes (FR)
(74) Mandataire: Hérard, Elise
(86) Numéro de dépôt international: PCT/EP2011/002295
(87) Numéro de publication internationale: WO 2011/138051

(56) Documents cités:
- WO-A1-2010/055273
- WO-A1-2010/089512
- WO-A2-2008/104722

## Description

L'invention vise un procédé de synthèse d'acides ou d'esters α,ω-amino-alcanoïques longue chaîne à partir d'un acide ou ester gras mono-insaturé comportant au moins une étape de métathèse.

L'industrie des polyamides que cela soit pour fabriquer des fibres synthétiques ou des résines thermodurcissables, utilise toute une gamme de monomères constitués par des diamines, des diacides et surtout des w-amino-acides à longue chaîne. Ces derniers sont usuellement appelés Nylon, définis par la longueur de chaîne méthylène (-CH₂)ₙ séparant deux fonctions amide -CO-NH-, C'est ainsi que sont connus les Nylon-6, Nylon 6-6, Nylon 6-10, Nylon 7, Nylon 8, Nylon 9, Nylon 11, Nylon 13, etc.

Ces monomères sont généralement fabriqués par voie de synthèse chimique en utilisant notamment comme matières premières des oléfines en C2 à C4, des cycloalcanes ou du benzène, hydrocarbures issus de sources fossiles, mais aussi dans certains cas particuliers à partir d'huile de ricin (Nylon 11) ou d'huile érucique (Nylon 13/13) ou lesquérolique (Nylon 13).

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Ce type d'approche n'a que peu d'exemples industriels. Un des rares exemples de procédé industriel utilisant un acide gras naturel comme matière première est celui de la fabrication à partir de l'acide ricinoléique extrait de l'huile de ricin, de l'acide amino-11-undécanoïque, qui est à la base de la synthèse du Rilsan 11 ^{®}. Ce procédé est décrit dans l'ouvrage « Les Procédés de Pétrochimie » de A. Chauvel et al. paru aux Editions TECHNIP (1986). L'acide amino-11-undécanoïque est obtenu en plusieurs étapes. La première consiste en une méthanolyse de l'huile de ricin en milieu basique produisant le ricinoléate de méthyle qui est ensuite soumis à une pyrolyse pour obtenir d'une part l'heptanaldéhyde et, d'autre part le undécylénate de méthyle. Ce dernier est passé en forme acide par hydrolyse. Ensuite l'acide formé est soumis à une hydrobromuration pour donner l'acide w-bromé d'où l'on passe par ammoniation à l'acide amino-11-undécanoïque.

Dans cette voie « bio », les principaux travaux ont porté sur la synthèse de l'acide amino-9-nonanoïque qui est le précurseur du Nylon 9 à partir de l'acide oléique d'origine naturelle.

En ce qui concerne ce monomère particulier on peut citer l'ouvrage « n-Nylons, Their Synthesis, Structure and Properties » - 1997 Ed.J. Wiley et Sons dont le chapitre 2.9 (pages 381 à 389) est consacré au Nylon 9. Cet article donne la synthèse des réalisations et des travaux conduits sur le sujet. On y mentionne, page 381, le procédé développé par l'ex Union Soviétique ayant conduit à la commercialisation du Pelargon®. On y mentionne également, page 384, un procédé développé au Japon utilisant l'acide oléique venant de l'huile de soja comme matière première. La description correspondante fait référence à l'ouvrage de A. Rawe « Organic Chemistry of Macromolecules » (1967) Marcel Dekker, Inc., dont la partie 15 est consacrée aux polyamides et qui mentionne en page 279 l'existence d'un tel procédé.

La demanderesse a pour sa part conduit des travaux dans ce domaine. Elle a décrit dans la demande de brevet français publiée sous le numéro FR 2912741 un procédé de synthèse de toute une gamme de ce type d'aminoacides/esters à partir d'un acide/ester gras naturel à longue chaîne en soumettant ce dernier à une réaction de métathèse catalytique croisée avec un composé insaturé comportant une fonction nitrile suivie d'une hydrogénation. Dans la demande de brevet français déposée le 17 novembre 2008 sous le numéro FR0857780 elle a également décrit un procédé de synthèse d'acides ω-amino-alcanoïques ou de leurs esters à partir d'acides gras naturels insaturés à longue chaîne transitant par un composé intermédiaire de type nitrile ω-insaturé dont l'une des variantes met en oeuvre dans la phase finale une métathèse croisée du nitrile w-insaturé avec un composé de type acrylate. Enfin dans la demande de brevet français déposée le 5 février 2009 sous le numéro FR0950704, elle a décrit une variante du procédé précédent dans laquelle le composé intermédiaire est du type dinitrile insaturé. L'ensemble de ces procédés conduit à une dernière étape d'hydrogénation de la fonction nitrile ainsi que de la double liaison.

Le procédé de l'invention a pour objet d'améliorer les performances des procédés mettant en oeuvre dans la phase finale successivement une métathèse croisée et une hydrogénation. Il est en effet important de pouvoir disposer d'une catalyse efficace de ces deux réactions successives tout en minimisant le nombre d'opérations qui a naturellement une incidence sur le coût final.

L'hydrogénation de la fonction nitrile en amine primaire est sur le plan industriel généralement réalisée au moyen de catalyseurs hétérogènes réducteurs forts tels que le cobalt ou le nickel de Raney. L'utilisation d'autres métaux connus pour leur activité catalytique en hydrogénation a également été envisagée dans des conditions hétérogènes. On peut citer par exemple le platine, le palladium, le ruthénium ou l'iridium seuls ou en combinaison. A titre d'illustration on peut citer le brevet UK 1 177 154 qui décrit l'utilisation de divers catalyseurs, platine, palladium et nickel de Raney pour l'hydrogénation de la fonction nitrile de l'acide 11-cyanoundécanoïque et le brevet UK 1 273 874 qui décrit l'utilisation d'un catalyseur au ruthénium déposé sur silice pour la même réaction conduisant à l'acide 12-aminodocécanoïque. Cependant le nickel déposé sur un support tel que la silice est le catalyseur le plus généralement adopté.

La catalyse homogène de cette réaction d'hydrogénation de nitriles en amines a également fait l'objet d'études et est décrite dans la littérature. On peut citer par exemple l'hydrogénation du benzonitrile en benzylamine avec des catalyseurs homogènes au ruthénium décrite par M. Hidai et al. dans Organometallics, 2002, 21, 3897 ainsi que l'article de R.H. Morris et al. dans Organometallics, 2007, 26, 5940-5949 ainsi que l' article de M. Beller et al. dans ChemSusChem 2008, 1, 1006; Chem. Eur. J. 2008, 14, 9491; Tetrahedron Lett. 2009,50, 3654. L'ajout d'une base pour réaliser ce type de réaction est décrit dans ces articles.

La séquence de réactions métathèse puis hydrogénation des produits de la métathèse est décrite dans la demande de brevet US 2009/048459 de la société Cargill qui décrit une méthode pour faire des produits hydrogénés de métathèse avec des étapes successives de métathèse et d'hydrogénation dans laquelle l'étape d'hydrogénation est réalisée en traitant le milieu réactionnel de métathèse, contenant le catalyseur de métathèse, au moyen d'un catalyseur d'hydrogénation hétérogène constitué selon tous les exemples par du nickel supporté. On peut également préciser que la réaction mise en oeuvre dans les exemples est une homométathèse d'huile de soja qui conduit en raison de la composition de cette huile et de l'absence de toute séparation des produits de la réaction de métathèse à un mélange complexe d'esters et non à un monomère susceptible d'être polymérisé.

Or la demanderesse a découvert qu'il était possible dans le procédé de conduire les étapes de métathèse et d'hydrogénation au moyen d'un seul composé catalytique initial comportant donc le même métal actif comme catalyseur. Le catalyseur de métathèse dégradé au terme de la réaction de métathèse remplit lors de la seconde étape la fonction de catalyseur d'hydrogénation.

L'invention a pour objet un procédé de synthèse d'un α,ω-aminoester (acide) saturé longue chaîne comportant de 6 à 17 atomes de carbone caractérisé en ce que l'on obtient dans une première étape par réaction de métathèse croisée entre un premier composé acrylique choisi parmi l'acrylonitrile, l'acide acrylique ou un ester acrylique et un second composé mono-insaturé comportant au moins une fonction trivalente, nitrile, acide ou ester, l'un de ces composés comportant une fonction nitrile et l'autre une fonction acide ou ester, en présence d'un catalyseur de métathèse de type carbènes de ruthénium et dans une deuxième étape par l'hydrogénation du nitrile-ester(acide) mono-insaturé obtenu, en présence du catalyseur de métathèse de l'étape précédente faisant fonction de catalyseur d'hydrogénation. La réaction de métathèse considérée est une réaction de métathèse croisée entre un composé acide ou ester mono-insaturé généralement issu de l'oléochimie avec l'acrylonitrile ou une réaction de métathèse croisée entre un composé nitrile insaturé généralement issu de l'oléochimie avec un composé acrylique, acide ou acrylate, et dans ce cas de préférence l'acrylate de méthyle.

Le procédé a été développé en vue de l'exploitation de matières premières issues de sources naturelles renouvelables. Il peut cependant aussi bien s'appliquer aux composés mono-insaturés analogues obtenus par synthèse chimique.

L'étape de métathèse est réalisée selon le schéma réactionnel suivant : 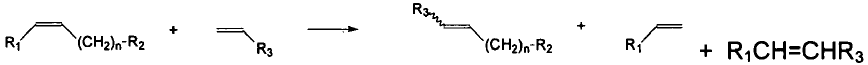
avec R₁=H ou (CH₂)ₘ-R₄,
R₂=COOR₅ ou CN,
R₃=COOR₅ ou CN,
R₄=H ou R₂,
R₅=radical alkyle de 1 à 4 atomes de carbone,
n=2 à 13,
m= 4 à 11 et,
R₂ différent de R₃.

La formule de l'α,ω-aminoester (acide) final synthétisé dépend essentiellement de celle du composé réagissant avec le composé acrylique.

Dans ce composé issu de l'oléochimie i.e. obtenu à partir d'esters ou acides gras naturels renouvelables, R₁ est soit H, soit un radical alkyle, soit un radical alkyle fonctionnel comportant une fonction trivalente (CN, COOH ou COOR).

R₁ sera H lorsque l'ester gras naturel sera par exemple soumis à une éthénolyse ou dans certains cas à une pyrolyse. La formule de l'α,ω-aminoester/acide obtenu est alors directement liée au radical -(CH₂)ₙ- de l'ester gras. C'est ainsi que n sera égal à 7 avec l'acide oléique, à 4 avec l'acide pétrosélénique, à 8 à partir d'acide ricinoléique soumis à une pyrolyse, à 10 à partir d'acide lesquérolique soumis à une pyrolyse etc..., ainsi que cela est décrit dans la demande de brevet français publiée sous le numéro FR 2 912 741.

R₁ sera un radical alkyle lorsque dans (CH₂)ₘ-R₄, R₄ est H. Ceci correspond à l'utilisation dans le procédé d'un acide gras naturel mono-insaturé tel que par exemple les acides oléique, palmitoléique, pétrosélénique, lauroléique etc...

R₁ sera un radical alkyle fonctionnel lorsque dans (CH₂)ₘ-R₄, R₄ est un radical représentant une fonction trivalente CN, COOH ou COOR qui sera identique à R₂. Le composé sera alors sous la forme diacide, diester ou dinitrile. Il sera alors particulièrement avantageux que la formule du composé présente une symétrie permettant d'optimiser les rendements en α,ω-aminoester/acide final. L'obtention de ce type de composés, par métathèse notamment, est décrite dans les demandes FR 2912741, FR0857780, et FR0950704 citées précédemment.

En ce qui concerne le composé acrylique, le choix de la fonction trivalente R₃ est lié à la nature de la fonction trivalente de l'autre composé, R₃ devant être nitrile lorsque R₂ est ester/acide et réciproquement ester/acide lorsque R₂ est nitrile.

Cette réaction conduit à des nitrile-acides ou des nitrile-esters insaturés.

De préférence, la réaction de métathèse croisée avec l'acrylonitrile est réalisée avec un composé choisi parmi l'acide 9-décénoïque ou le 9-décénoate de méthyle, issus de l'éthénolyse de l'acide oléique ou de l'oléate de méthyle, l'acide 10-undécénoïque ou le 10-undécénoate de méthyle, issus du craquage de l'acide ricinoléique ou du ricinoléate de méthyle, l'acide oléique ou l'oléate de méthyle, l'acide 9-octadécènedioïque ou le 9-octadécènedioate de méthyle, issus de l'homométathèse ou de la fermentation de l'acide oléique, l'acide érucique et l'érucate de méthyle, l'acide 12-tridécénoïque ou le 12-tridécénoate de méthyle issus de l'acide lesquérolique.
la réaction de métathèse croisée de l'ester(acide) acrylique est réalisée avec un composé choisi parmi le 9-décènenitrile issu de l'acide 9-décénoïque, le 10-undécènenitrile issu de l'acide 10-undécénoïque, le 9-octadécènenitrile ou oléonitrile, issu de l'acide oléique, le 9-octadécènedinitrile, issu de l'acide 9-octadécènedioïque, l'éruconitrile, le 12-tridécénonitrile issu de l'acide lesquérolique.

La réaction de métathèse croisée avec un composé de type acrylique est réalisée dans des conditions parfaitement connues. La réaction de métathèse est réalisée de préférence à une température de réaction comprise entre 20 et 120 °C et la pression est comprise entre 1 et 30 bars en présence d'un catalyseur à base de ruthénium. Elle sera de préférence conduite à basse pression comprise entre 1 et 10 bars et de façon plus préférée à la pression atmosphérique lorsque la métathèse croisée conduit à la formation d'un composé léger par exemple de l'éthylène pour en permettre un dégagement aisé. La réaction peut être conduite sans solvant ou en présence d'un solvant comme le toluène, les xylènes ou le dichlorométhane. le benzene, le chlorobenzene ou le dimethylcarbonate.

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628.

Plus récemment, sont apparus les catalyseurs dits de Grubbs (Grubbs et al. Angew. Chem., Ed. Engl. 34 (1995) 2039 et Organic Letters 1 (1999) 953) qui sont des complexes ruthénium-benzylidène opérant en catalyse homogène.

Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

Les catalyseurs au ruthénium sont choisis de préférence parmi les catalyseurs chargés ou non-chargés de formule générale :

(X1)ₐ (X2)_{b}Ru(carbène C) (L1 )_{c}(L2)_{d} (L3)ₑ

dans laquelle :
- a, b, c, d et e sont des nombres entiers, identiques ou différents, avec a et b égaux à 0, 1 ou 2 ; c,d et e égaux à 0, 1, 2, 3 ou 4;
- X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés. X1 ou X2 peuvent être liés à (L1 ou L2) ou au (carbène C) de façon à former un ligand bidenté ou chélate sur le ruthénium ; et
- L1, L2 et L3 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique

L1, L2 ou L3 peuvent être liés au (carbène C) de façon à former un ligand bidenté ou chélate, ou tridenté

Le (carbène C) étant représenté par la formule générale : C_(R1)_(R2) pour laquelle R1 et R2 sont des groupes identiques ou différents tels que l'hydrogène ou tout autre groupe hydrocarboné saturé ou insaturé, cyclique, branché ou linéaire, ou aromatique. A titre d'exemples, on pourra citer les complexes du ruthénium alkylidènes, benzylidène ou cumulènes tels que les vinylidènes Ru=C=CHR ou allénylidènes Ru=C=C=CR1R2 ou indénylidènes.

Un groupe fonctionnel permettant d'améliorer la rétention du complexe du ruthénium dans un liquide ionique peut être greffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, N°2, 2007, p.45-52. Les catalyseurs préférés sont le catalyseur Umicore M51 (commercialisé par la société Umicore) de formule (A) ci-dessous, et le catalyseur Hoveyda-Grubbs de 2^{nd} génération dénommé aussi Hoveyda II (commercialisé par Sigma-Aldrich) de formule (B) ci-dessous.

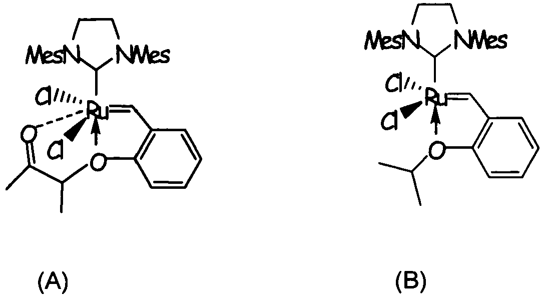

Le temps de réaction est choisi en fonction des réactifs et des conditions opératoires mis en oeuvre et pour atteindre le terme de la réaction.

La métathèse étant une réaction équilibrée, il convient de déplacer cet équilibre pour aller vers la conversion totale. Pour ce faire dans le cas où le co-produit de la réaction est une oléfine légère telle que l'éthylène, il est aisé de « dégazer » le réacteur de temps en temps pour forcer l'élimination des légers et donc aller à conversion totale. Dans le cas où le co-produit est une oléfine plus lourde, éventuellement fonctionnelle, l'opération d'extraction est plus délicate dans la mesure où il faut maintenir dans le milieu réactionnel, les deux réactifs et le catalyseur. Par ailleurs si on doit séparer au moins en partie le nitrile-ester(acide) insaturé par distillation et éliminer les légers avant l'hydrogénation, il faut opérer de sorte que le catalyseur de métathèse reste dans la fraction lourde avec le nitrile-ester(acide) pour l'utiliser dans sa fonction de catalyseur d'hydrogénation. Dans cette opération lors de la séparation on n'élimine pas du milieu les composés très lourds qui seront donc hydrogénés avec la fraction lourde, leur séparation intervenant lors d'une purification ultérieure de l'amino-acide/ester final.

L'autre façon de déplacer l'équilibre est d'utiliser un excès de réactif, typiquement ici un excès d'acrylonitrile ou d'acrylate d'alkyle (de méthyle en général). D'un point de vue procédé, la première étape serait conduite à son terme avec l'épuisement du catalyseur de métathèse, l'excès d'acrylate ou d'acrylonitrile serait distillé pour recyclage, puis en seconde étape le composé α-ω nitrile-ester/acide insaturé contenu dans le milieu réactionnel serait hydrogéné en présence du métal du catalyseur de 1^{ère} étape dans sa fonction hydrogénation.

La quantité de catalyseur de métathèse au ruthénium introduite lors de la première étape est choisie de telle sorte qu'il assure toute la transformation possible du réactif non acrylique contenu dans la charge. On observe que ledit catalyseur, dans les conditions opérationnelles de l'étape de métathèse, est au terme de la réaction transformé ; il s'épuise ou se désactive et perd son activité catalytique en terme de métathèse - il sera désigné par la suite par le qualificatif de « dégradé » pour ladite réaction. En procédé batch, la quantité de catalyseur peut aisément être réglée pour donner la conversion souhaitée à dégradation totale du catalyseur.

Au terme de l'étape de métathèse, le milieu réactionnel contenant le ruthénium est donc soumis à une hydrogénation. Le catalyseur de métathèse au ruthénium est dégradé à l'issue de l'étape de métathèse, mais le métal est toujours présent dans le milieu réactionnel sous une forme appropriée pour l'étape d'hydrogénation.

La réaction d'hydrogénation est ainsi réalisée directement sur le mélange réactionnel issu de l'étape de métathèse et en présence du catalyseur de métathèse résiduel faisant fonction de catalyseur d'hydrogénation, sous pression d'hydrogène et en présence d'une base. La pression est comprise entre 5 et 100 bars, de préférence entre 20 et 30 bars. La température est comprise entre 50 et 150°C, de préférence entre 80 et 100°C. La base peut être par exemple la soude, la potasse, le tertiobutylate de potassium ou l'ammoniac. La base est généralement utilisée à une teneur de 10 à 80% en mole par rapport au substrat nitrile-ester insaturé.

La réaction d'hydrogénation peut être réalisée avec ou sans solvant. Dans le cas d'une réaction en milieu solvant, les solvants préférés utilisés pour les étapes de métathèse et d'hydrogénation sont les solvants aromatiques comme le toluène ou les xylènes ou un solvant chloré comme le dichlorométhanen le chlorobenzene, le dimethylcarbonate.

A l'issue de cette étape d'hydrogénation réalisée sans catalyseur spécifique d'hydrogénation, le taux de transformation de la fonction nitrile en amine primaire est particulièrement élevé même sans addition de NH3 ainsi bien entendu que la réduction de l'insaturation oléfinique sans que la fonction carboxyle ait été touchée.

Il est ainsi montré de façon inattendue que le catalyseur dégradé de métathèse présentait activité et sélectivité pour l'hydrogénation des nitrile-acides ou nitrile-esters insaturés en amino-acides ou amino-esters saturés.

Le catalyseur dégradé de métathèse peut éventuellement être mis en oeuvre avec en complément un catalyseur classique d'hydrogénation pour l'étape d'hydrogénation. Parmi les métaux utilisés classiquement pour l'hydrogénation, on peut citer le nickel, le palladium, le platine, le rhodium ou l'iridium. De préférence, le catalyseur dégradé de métathèse pourrait être complété par du nickel de Raney ou du palladium sur charbon.

Ainsi, dans un mode de réalisation particulier la réaction d'hydrogénation est réalisée en présence du catalyseur dégradé de métathèse issu de la première étape complété par un catalyseur d'hydrogénation conventionnel.

Il peut également être mis en oeuvre en présence d'un support solide (charbon, SiC...) afin de simplifier sa récupération.

Les amino-acides ou amino-esters obtenus selon le procédé de l'invention peuvent être utilisés comme monomères pour la synthèse de polyamides.

Le procédé de l'invention est illustré par les exemples suivants.

### Exemple 1

Métathèse croisée de l'undécénoate de méthyle avec l'acrylonitrile suivie d'hydrogénation avec le catalyseur de Hoveyda-Grubbs II :

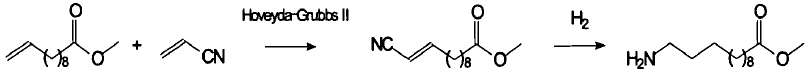

Dans un tube de Schlenk de 50ml purgé à l'azote, on charge 100mg de 10-undécénoate de méthyle (0,5 mmol), 53mg d'acrylonitrile (1 mmol) et 10 ml de toluène distillé sur sodium-benzophénone. On ajoute 9,5 mg de catalyseur de Hoveyda-Grubbs de 2^{nd} génération (1,5.10⁻² mmol - fournisseur Sigma-Aldrich) et on chauffe à 100°C pendant 4 heures.

L'analyse par chromatographie en phase gaz montre que la conversion du 10-undécénoate de méthyle est de 100% (96%) molaire et que le rendement en nitrile-ester insaturé est de 95% molaire.

Le mélange réactionnel est alors transféré dans une bombe de Parr de 50ml (22mL). On ajoute 17mg de potasse (0,3 mmol) et on pressurise sous 20 bars d'hydrogène. Sous agitation magnétique, on chauffe à 80°C pendant 48h.

L'analyse par chromatographie en phase gaz montre que la conversion du nitrile-ester insaturé est de 100% molaire et que le rendement en 12-amino-dodécanoate de méthyle est de 90% molaire.

### Exemple 2

Métathèse croisée de l'undécénoate de méthyle avec l'acrylonitrile suivie d'hygrogénation avec catalyseur Umicore M51 :
Le mode opératoire est le même que dans l'exemple 1, le catalyseur Hoveyda-Grubbs II étant remplacé par 10mg de catalyseur Umicore M51 (1,5.10⁻² mmol - fournisseur Umicore), et la potasse étant remplacée par 8,5mg de tertiobutylate de potassium (0,075 mmol).

L'analyse par chromatographie en phase gaz montre que le rendement en 12-amino-dodécanoate de méthyle est de 88% molaire.

### Exemple 3

Métathèse croisée undécénenitrile/acrylate de méthyle suivie d'hydrogénation avec le catalyseur de Hoveyda-Grubbs II :

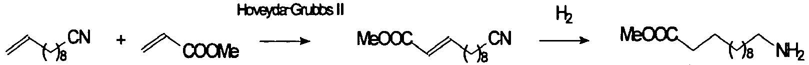

Dans un tube de Schlenk de 50ml purgé à l'azote, on charge 83mg de 10-undécènenitrile (0,5 mmol), 86mg d'acrylate de méthyle (1 mmol) et 10 ml de toluène distillé sur sodium benzophénone. On ajoute 9,5 mg de catalyseur de Hoveyda-Grubbs de 2^{nd} génération (1,5.10⁻² mmol) et on chauffe à 100°C pendant 1 heure.

L'analyse par chromatographie en phase gaz montre que la conversion du 10-undécènitrile est de 100% et que le rendement en nitrile-ester insaturé est de 98%.

Le mélange réactionnel est alors transféré dans une bombe de Parr de 50ml (22mL). On ajoute 17mg de tertiobutylate de potassium (0,15 mmol) et on pressurise sous 20 bars d'hydrogène. Sous agitation magnétique, on chauffe à 80°C pendant 40h.

L'analyse par chromatographie en phase gaz montre que la conversion du nitrile-ester insaturé est de 100% molaire et que le rendement en 12-amino-dodécanoate de méthyle est de 90% molaire.

### Exemple 4

Métathèse croisée undécénoate de méthyle/acrylonitrile avec catalyseur de Hoveyda-Grubbs II suivie d'hydrogénation avec ajout complémentaire de catalyseur Pd/C :
Dans un tube de Schlenk de 50ml purgé à l'azote, on charge 100mg de 10-undécénoate de méthyle (0,5 mmol), 53mg d'acrylonitrile (1 mmol) et 10 ml de toluène distillé sur sodium benzophénone. On ajoute 3mg de catalyseur de Hoveyda-Grubbs de 2^{nd} génération (5.10⁻³ mmol) et on chauffe à 100°C pendant 4 heures.

L'analyse par chromatographie en phase gaz montre que la conversion du 10-undécénoate de méthyle est de 98% et que le rendement en nitrile-ester insaturé est de 93%.

Le mélange réactionnel est alors transféré dans une bombe de Parr de 50ml (22mL). On ajoute 10mg de catalyseur 1% Pd/C et 17mg de tertiobutylate de potassium (0,15 mmol) et on pressurise sous 20 bars d'hydrogène. Sous agitation magnétique, on chauffe à 80°C pendant 48h.

L'analyse par chromatographie en phase gaz montre que la conversion du nitrile-ester insaturé est de 90% et que le rendement en 12-amino-dodécanoate de méthyle est de 64%.

## Revendications

1. Procédé de synthèse d'un α,ω-aminoester (acide) saturé longue chaîne comportant de 6 à 17 atomes de carbone, **caractérisé en ce que** l'on obtient dans une première étape un nitrile-ester(acide) mono-insaturé par réaction de métathèse croisée entre un premier composé acrylique choisi parmi l'acrylonitrile, l'acide acrylique ou un ester acrylique et un second composé mono-insaturé comportant au moins une fonction trivalente, nitrile, acide ou ester, l'un de ces composés comportant une fonction nitrile et l'autre une fonction acide ou ester, en présence d'un catalyseur de métathèse de type carbènes de ruthénium et dans une deuxième étape on obtient l'α,ω-aminoester (acide) saturé longue chaîne par l'hydrogénation du nitrile-ester(acide) mono-insaturé obtenu en présence du catalyseur de métathèse de l'étape précédente faisant fonction de catalyseur d'hydrogénation.

2. Procédé selon la revendication 1 **caractérisé en ce que** l'étape de métathèse est réalisée selon le schéma réactionnel suivant 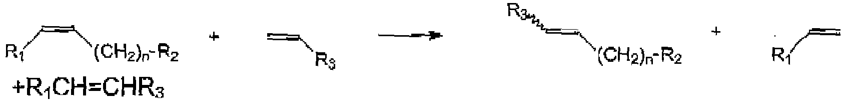 avec
R₁=H ou (CH₂)ₘ-R₄,
R₂=COOR₅ ou CN,
R₃=COOR₅ ou CN,
R₄=H ou R₂,
R₅=radical alkyle de 1 à 4 atomes de carbone,
n=2 à 13, m= 4 à 11 et,
R2 différent de R3.

3. Procédé selon l'une des revendications 1 et 2 **caractérisé en ce que** la réaction de métathèse croisée avec l'acrylonitrile est réalisée avec un composé choisi parmi l'acide 9-décénoïque, le 9-décénoate de méthyle, l'acide 10-undécénoïque, le 10-undécénoate de méthyle, l'acide oléique, l'oléate de méthyle, l'acide 9-octadécènedioïque, le 9-octadécènedioate de méthyle, l'acide érucique, l'érucate de méthyle, l'acide 12-tridécénoïque et le 12-tridécénoate de méthyle.

4. Procédé selon l'une des revendications 1 et 2 **caractérisé en ce que** la réaction de métathèse croisée de l'ester(acide) acrylique est réalisée avec un composé choisi parmi le 9-décènenitrile, le 10-undécènenitrile, le 9-octadécènenitrile ou olélonitrile, le 9-octadécènedinitrile, l'éruconitrile, le 12-tridécénonitrile.

5. Procédé selon l'une des revendications 1 à 4 **caractérisé en ce que** la réaction de métathèse est réalisée à une température de réaction comprise entre 20 et 120 °C et sous une pression comprise entre 1 et 30 bars et de préférence comprise entre 1 et 10 bars.

6. Procédé selon l'une des revendications 1 à 5 **caractérisé en ce que** la réaction de métathèse est réalisée en présence d'un catalyseur au ruthénium choisi parmi les catalyseurs chargés ou non-chargés de formule générale :(X1)ₐ (X2)_{b}Ru(carbène C) (L1)_{c}(L2)_{d},(L3)ₑ dans laquelle :
• a, b, c, d et e sont des nombres entiers, identiques ou différents, avec a et b égaux à 0, 1 ou 2 ; c, d et e égaux à 0,1, 2, 3 ou 4 ;
• X1 et X2, identiques ou différents, représentent chacun un ligand mono- ou multi-chélatant, chargé ou non, de préférence choisi parmi les halogénures, le sulfate, le carbonate, les carboxylates, les alcoolates, les phénolates, les amidures, le tosylate, l'hexafluorophosphate, le tétrafluorabarate, le bis-triflylamidure, le tétraphénylborate et dérivés. X1 ou X2 peuvent être liés à Y1 ou Y2 (L1 ou L2) ou au (carbène C) de façon à former un ligand bidenté ou chélate sur le ruthénium ; et
• L1, L2, et L3 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique
L1, L2 ou L3 pouvant être liés au (carbène C) de façon à former un ligand bidenté ou chélate, ou tridenté le (carbène C) étant représenté par la formule générale : C_(R1)_(R2) pour laquelle R1 et R2 sont des groupes identiques ou différents de préférence l'hydrogène ou tout autre groupe hydrocarbonate saturé ou insaturé, cyclique, branché ou linéaire, ou aromatique.

7. Procédé selon la revendication 6 **caractérisé en ce que** le catalyseur de métathèse répond à l'une des formules (A) et (B) ci-dessous

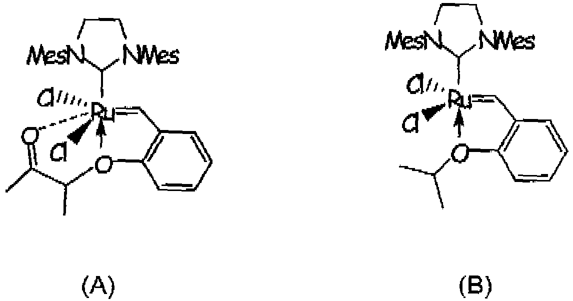

8. Procédé selon l'une des revendications 1 à 7 **caractérisé en ce que** la réaction d'hydrogénation est réalisée sur le mélange réactionnel issu de l'étape de métathèse et en présence du catalyseur de métathèse résiduel faisant fonction de catalyseur d'hydrogénation, sous pression d'hydrogène et en présence d'une base.

9. Procédé selon l'une des revendications 1 à 8 **caractérisé en ce que** la réaction d'hydrogénation est réalisée à une pression comprise 5 et 100 bars, de préférence entre 20 et 30 bars et à une température comprise entre 50 et 150°C, de préférence entre 80 et 100°C.

10. Procédé selon l'une des revendications 1 à 9 **caractérisé en ce que** la réaction d'hydrogénation est réalisée en présence d'une base choisie parmi la soude, la potasse, le tertiobutylate de potassium ou l'ammoniac, à une teneur de 10 à 80% en mole par rapport au substrat nitrile-ester insaturé.

11. Procédé selon l'une des revendications 1 à 10 **caractérisé en ce que** la réaction d'hydrogénation est réalisée en présence du catalyseur dégradé de métathèse issu de la première étape complété par un catalyseur d'hydrogénation conventionnel.

## Patentansprüche

1. Verfahren zur Synthese eines gesättigten langkettigen α,ω-Aminoesters (einer gesättigten langkettigen α,ω-Aminosäure) mit 6 bis 17 Kohlenstoffatomen, **dadurch gekennzeichnet, dass** man in einem ersten Schritt durch Kreuzmetathesereaktion zwischen einer ersten Acrylverbindung, die aus Acrylnitril, Acrylsäure oder einem Acrylsäureester ausgewählt wird, und einer zweiten einfach ungesättigten Verbindung mit mindestens einer dreiwertigen Nitril-, Säure- oder Esterfunktion, wobei eine dieser Verbindungen eine Nitrilfunktion umfasst und die andere eine Säure- oder Esterfunktion umfasst, in Gegenwart eines Metathesekatalysators vom Rutheniumcarben-Typ einen einfach ungesättigten Nitrilester (eine einfach ungesättigte Nitrilsäure) erhält und in einem zweiten Schritt durch Hydrierung des erhaltenen einfach ungesättigten Nitrilesters (der erhaltenen einfach ungesättigten Nitrilsäure) in Gegenwart des Metathesekatalysators des vorhergehenden Schritts, der als Hydrierkatalysator fungiert, den gesättigten langkettigen α,ω-Aminoester (die gesättigte langkettige α,ω-Aminosäure) erhält.

2. Verfahren nach Anspruch 1, **dadurch gekennzeichnet, dass** der Metatheseschritt gemäß dem folgenden Reaktionsschema durchgeführt wird: 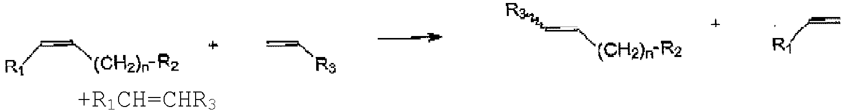 wobei
R₁ = H oder (CH₂)ₘ-R₄,
R₂ = COOR₅ oder CN,
R₃ = COOR₅ oder CN,
R₄ = H oder R₂,
R₅ = Alkylrest mit 1 bis 4 Kohlenstoffatomen,
n = 2 bis 13, m = 4 bis 11, und
R₂ von R₃ verschieden ist.

3. Verfahren nach einem der Ansprüche 1 und 2, **dadurch gekennzeichnet, dass** die Kreuzmetathesereaktion mit Acrylnitril mit einer aus 9-Decensäure, 9-Decensäuremethylester, 10-Undecensäure, 10-Undecensäuremethylester, Ölsäure, Ölsäuremethylester, 9-Octadecendisäure, 9-Octadecendisäuremethylester, Erucasäure, Erucasäuremethylester, 12-Tridecensäure und 12-Tridecensäuremethylester ausgewählten Verbindung durchgeführt wird.

4. Verfahren nach einem der Ansprüche 1 und 2, **dadurch gekennzeichnet, dass** die Kreuzmetathesereaktion des Acrylsäureesters (der Acrylsäure) mit einer aus 9-Decennitril, 10-Undecennitril, 9-Octadecennitril oder Ölsäurenitril, 9-Octadecendinitril, Erucasäurenitril oder 12-Tridecensäurenitril ausgewählten Verbindung durchgeführt wird.

5. Verfahren nach einem der Ansprüche 1 bis 4, **dadurch gekennzeichnet, dass** die Metathesereaktion bei einer Reaktionstemperatur zwischen 20 und 120°C und unter einem Druck zwischen 1 und 30 bar und vorzugsweise zwischen 1 und 10 bar durchgeführt wird.

6. Verfahren nach einem der Ansprüche 1 bis 5, **dadurch gekennzeichnet, dass** die Metathesereaktion in Gegenwart eines aus geladenen oder ungeladenen Katalysatoren der allgemeinen Formel (X1)ₐ(X2)_{b}Ru (Carben C) (L1)_{c}(L2)_{d}(L3)ₑ ausgewählten Rutheniumkatalysators durchgeführt wird, wobei:
• a, b, c, d und e gleiche oder verschiedene ganze Zahlen sind, wobei a und b gleich 0, 1 oder 2 sind und c, d und e gleich 0, 1, 2, 3 oder 4 sind;
• X1 und X2 gleich oder verschieden sind und jeweils für einen geladenen oder ungeladenen Mono- oder Multichelatliganden stehen, der vorzugsweise aus Halogeniden, Sulfat, Carbonat, Carboxylaten, Alkoxiden, Phenolaten, Amiden, Tosylat, Hexafluorophosphat, Tetrafluoroborat, Bis(triflyl)amid, Tetraphenylborat und Derivaten ausgewählt ist, wobei X1 und X2 an Y1 oder Y2 (L1 oder L2) oder an (Carben C) gebunden sein können, so dass sich ein zweizähniger Ligand oder Chelatligand am Ruthenium ergibt, und
• L1, L2 und L3 gleich oder verschieden sind und für elektronenliefernde Liganden wie Phosphin, Phosphit, Phosphonit, Phosphinit, Arsin, Stilbin, ein Olefin oder einen Aromaten, eine Carbonylverbindung, einen Ether, einen Alkohol, ein Amin, ein Pyridin oder Pyridinderivat, ein Imin, einen Thioether oder ein heterocyclisches Carben stehen,
wobei L1, L2 oder L3 an (Carben C) gebunden sein können, so dass sich ein zweizähniger Ligand oder Chelatligand oder dreizähniger Ligand ergibt, wobei das (Carben C) durch die folgende allgemeine Formel wiedergegeben wird: C_(R1)_(R2), wobei R1 und R2 gleiche oder verschiedene Gruppen sind, vorzugsweise Wasserstoff oder eine beliebige andere gesättigte oder ungesättigte cyclische, verzweigte oder lineare oder aromatische Hydrocarbonylgruppe.

7. Verfahren nach Anspruch 6, **dadurch gekennzeichnet, dass** der Metathesekatalysator einer der nachstehenden Formeln (A) und (B) entspricht:

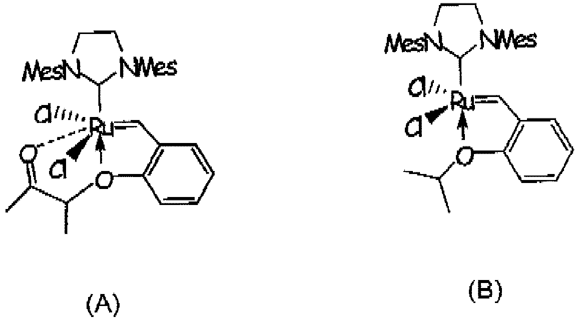

8. Verfahren nach einem der Ansprüche 1 bis 7, **dadurch gekennzeichnet, dass** die Hydrierungsreaktion an der Reaktionsmischung aus dem Metatheseschritt und in Gegenwart des restlichen Metathesekatalysators, der als Hydrierkatalysator fungiert, unter Wasserstoffdruck und in Gegenwart einer Base durchgeführt wird.

9. Verfahren nach einem der Ansprüche 1 bis 8, **dadurch gekennzeichnet, dass** die Hydrierungsreaktion bei einem Druck von 5 und 100 bar, vorzugsweise zwischen 20 und 30 bar, und einer Temperatur zwischen 50 und 150°C, vorzugsweise zwischen 80 und 100°C, durchgeführt wird.

10. Verfahren nach einem der Ansprüche 1 bis 9, **dadurch gekennzeichnet, dass** die Hydrierungsreaktion in Gegenwart einer aus Natriumhydroxid, Kaliumhydroxid, Kalium-tert-butylat oder Ammoniak ausgewählten Base in einem Gehalt von 10 bis 80 Mol-%, bezogen auf das ungesättigte Nitrilester-Substrat, durchgeführt wird.

11. Verfahren nach einem der Ansprüche 1 bis 10, **dadurch gekennzeichnet, dass** die Hydrierungsreaktion in Gegenwart des abgebauten Metathesekatalysators aus dem ersten Schritt ergänzt durch einen herkömmlichen Hydrierkatalysator durchgeführt wird.

## Claims

1. Process for the synthesis of a saturated long-chain α,ω-amino ester (acid) comprising from 6 to 17 carbon atoms, **characterized in that** a monounsaturated nitrile-ester (acid) is obtained, in a first stage, by a cross metathesis reaction between a first acrylic compound, chosen from acrylonitrile, acrylic acid or an acrylic ester, and a second monounsaturated compound comprising at least one nitrile, acid or ester trivalent functional group, one of these compounds comprising a nitrile functional group and the other an acid or ester functional group, in the presence of a metathesis catalyst of ruthenium carbenes type, and, in a second stage, the saturated long-chain α,ω-amino ester (acid) is obtained by the hydrogenation of the monounsaturated nitrile-ester (acid) obtained in the presence of the metathesis catalyst from the preceding stage acting as hydrogenation catalyst.

2. Process according to Claim 1, **characterized in that** the metathesis stage is carried out according to the following reaction scheme: 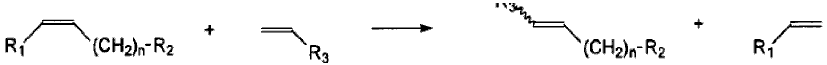 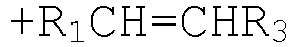 with
R₁ = H or (CH₂)ₘ-R₄,
R₂ = COOR₅ or CN,
R₃ = COOR₅ or CN,
R₄ = H or R₂,
R₅ = alkyl radical of 1 to 4 carbon atoms,
n = 2 to 13, m = 4 to 11, and
R₂ is different from R₃.

3. Process according to either of Claims 1 and 2, **characterized in that** the cross metathesis reaction with acrylonitrile is carried out with a compound chosen from 9-decenoic acid, methyl 9-decenoate, 10-undecenoic acid, methyl 10-undecenoate, oleic acid, methyl oleate, 9-octadecenedioic acid, methyl 9-octadecenedioate, erucic acid, methyl erucate, 12-tridecenoic acid and methyl 12-tridecenoate.

4. Process according to either of Claims 1 and 2, **characterized in that** the cross metathesis reaction of the acrylic ester (acid) is carried out with a compound chosen from 9-decenenitrile, 10-undecenenitrile, 9-octadecenenitrile or oleonitrile, 9-octadecenedinitrile, eruconitrile or 12-tridecenonitrile.

5. Process according to one of Claims 1 to 4, **characterized in that** the metathesis reaction is carried out at a reaction temperature of between 20 and 120°C and under a pressure of between 1 and 30 bar and preferably of between 1 and 10 bar.

6. Process according to one of Claims 1 to 5, **characterized in that** the metathesis reaction is carried out in the presence of a ruthenium catalyst chosen from charged or uncharged catalysts of general formula:
(X1)ₐ(X2)_{b}Ru(carbene C) (L1)_{c}(L2)_{d}(L3)ₑ
in which:
• a, b, c, d and e are identical or different integers, with a and b equal to 0, 1 or 2; c, d and e equal to 0, 1, 2, 3 or 4;
• X1 and X2, which are identical or different, each represent a charged or uncharged and monochelating or polychelating ligand, preferably chosen from halides, sulfate, carbonate, carboxylates, alkoxides, phenolates, amides, tosylate, hexafluorophosphate, tetrafluoroborate, bis(triflyl)amide, tetraphenylborate and derivatives. X1 or X2 can be bonded to Y1 or Y2 (L1 or L2) or to the (carbene C) so as to form a bidentate or chelate ligand on the ruthenium; and
• L1, L2 and L3, which are identical or different, are electron-donating ligands, such as phosphine, phosphite, phosphonite, phosphinite, arsine, stilbene, an olefin or an aromatic compound, a carbonyl compound, an ether, an alcohol, an amine, a pyridine or derivative, an imine, a thioether or a heterocyclic carbene;
it being possible for L1, L2 or L3 to be bonded to the (carbene C) so as to form a bidentate or chelate ligand, or a tridentate ligand, the (carbene C) being represented by the general formula: C_(R1)_(R2), for which R1 and R2 are identical or different groups, preferably hydrogen or any other saturated or unsaturated and cyclic, branched or linear hydrocarbonyl group or aromatic hydrocarbonyl group.

7. Process according to Claim 6, **characterized in that** the metathesis catalyst corresponds to either of the formulae (A) and (B) below:

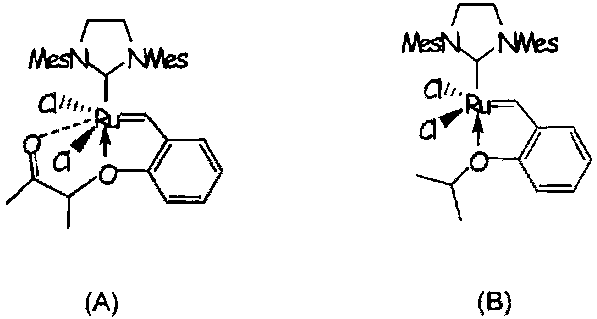

8. Process according to one of Claims 1 to 7, **characterized in that** the hydrogenation reaction is carried out on the reaction mixture resulting from the metathesis stage and in the presence of the residual metathesis catalyst acting as hydrogenation catalyst, under a hydrogen pressure and in the presence of a base.

9. Process according to one of Claims 1 to 8, **characterized in that** the hydrogenation reaction is carried out at a pressure of 5 and 100 bar, preferably between 20 and 30 bar, and at a temperature of between 50 and 150°C, preferably between 80 and 100°C.

10. Process according to one of Claims 1 to 9, **characterized in that** the hydrogenation reaction is carried out in the presence of a base chosen from sodium hydroxide, potassium hydroxide, potassium tert-butoxide or ammonia, at a content of 10 to 80 mol% with respect to the unsaturated nitrile-ester substrate.

11. Process according to one of Claims 1 to 10, **characterized in that** the hydrogenation reaction is carried out in the presence of the degraded metathesis catalyst resulting from the first stage supplemented by a conventional hydrogenation catalyst.
